# FASCICULE DE BREVET EUROPEEN

(11) **EP 1 358 487 B1**
(45) Date de publication et mention de la délivrance du brevet: **26.08.2009**
(21) Numéro de dépôt: 02702458.7
(22) Date de dépôt: 06.02.2002
(51) Int. Cl.: G01N 35/08, G01J 3/42, G01N 21/31

(54) **DISPOSITIF D'ANALYSE PAR SPECTROPHOTOMETRIE**
VORRICHTUNG ZUR ANALYSE DURCH SPEKTROPHOTOMETRIE
SPECTROPHOTOMETRY ANALYSIS DEVICE

(30) Priorité: 08.02.2001 FR 0101740
(43) Date de publication de la demande: 05.11.2003
(73) Titulaire: Microdom, 95157 Taverny Cedex (FR)
(72) Inventeur: THERRY, Francis, F-95 650 MONTGEROULT (FR); LEBOEUF, Jean-Pierre, F-95 590 PRESLES (FR)
(74) Mandataire: Camus, Olivier Jean-Claude
(86) Numéro de dépôt international: PCT/FR2002/000469
(87) Numéro de publication internationale: WO 2002/063309

(56) Documents cités:
- EP-B- 0 624 245
- WO-A-99/08115
- DE-A- 10 002 106
- DUNN B C ET AL: "STOPPED-FLOW RAPID-SCAN FOURIER TRANSFORM INFRARED SPECTROSCOPY" APPLIED SPECTROSCOPY, THE SOCIETY FOR APPLIED SPECTROSCOPY. BALTIMORE, US, vol. 53, no. 3, mars 1999 (1999-03), pages 292-296, XP000805989 ISSN: 0003-7028

## Description

La présente invention a pour objet un dispositif d'analyse par spectrophotométrie. Elle trouve plus particulièrement son utilisation dans le domaine des analyses de fluides et de liquides, notamment pour connaître des concentrations de différents métabolites contenus dans ces liquides. Les liquides pouvant être analysés par ce type de dispositif sont, par exemple, des boissons alcoolisées ou non, ou des liquides issus de corps humains ou animaux tels que du sang. Avec ces méthodes, on obtient un dosage quantitatif des composants chimiques présents dans les liquides. Dans l'état de la technique, on connaît un appareil de dosage spectrophotométrique de liquide aqueux mettant en oeuvre un spectrophotomètre faisant l'acquisition de spectres d'absorbance du liquide analysé pour des longueurs d'onde émises dans le domaine proche et moyen infrarouge. L'intérêt de l'invention est de permettre un dosage quantitatif plus fin et plus vaste des molécules présentes dans le fluide à analyser.

Dans l'état de la technique, on connaît pour effectuer un dosage fin et exhaustif des composants chimiques contenus dans un fluide, une méthode mettant en oeuvre un spectromètre de masse. Mais un tel appareil coûte très cher. Il ne peut donc pas être acheté à grande échelle pour réaliser de nombreux dosages quantitatifs.

Dans l'état de la technique, on connaît un procédé et un appareil de dosage spectrophotométrique des liquides aqueux, par exemple décrit dans le document WO-A-92/22803, mettant en oeuvre un dosage effectué par spectrophotométrie dans une plage proche ou moyenne infrarouge dont la longueur d'onde est comprise entre 2 et 25 micromètres. Un tel procédé permet de déterminer des concentrations de composants contenus dans le liquide aqueux analysé.

Le spectrophotomètre permet d'obtenir un interférogramme ou spectre d'absorbance pour des gammes de longueurs d'onde émises par cet appareil. Le spectre d'absorbance correspond aux longueurs d'onde absorbées dans le liquide analysé. Pour doser des composants d'un liquide à tester, on compare un interférogramme du liquide testé avec un interférogramme de référence obtenu avec le même appareil avec un liquide de référence. Par exemple, on peut utiliser une méthode mathématique mettant en oeuvre des régressions linéaires multiples (MLR). Dans ce cas, le liquide de référence comporte des concentrations connues des composants à doser. Ensuite, pour déterminer des concentrations de ces composants dans le liquide à tester, on établit un système d'équations. Les concentrations sont connues par l'intermédiaire de méthodes tierces. Dans une variante, on peut également déterminer les dosages des différents composants en appliquant une méthode de résolution mathématique des moindres carrés partiels (PLS). Cette méthode mathématique nécessite une acquisition d'un nombre fini de spectres, les spectres les plus représentatifs étant utilisés pour le calcul matriciel.

Chaque composant contenu dans un liquide à tester est préférentiellement dosé par l'étude d'un spectre d'absorbance pour une gamme de longueurs d'onde donnée. Ces gammes de longueurs d'onde peuvent être différentes selon les composants. En effet, on choisit de manière préférentielle la gamme de longueurs d'onde permettant d'obtenir la plus grande précision de mesure pour chacun des composants.

Cependant dans l'état de la technique, on connaît uniquement des appareils de spectrophotométrie dans le proche ou le moyen infrarouge balayant un spectre de longueurs d'onde allant généralement au plus vaste de 2 microns à 10 microns, voire 25 microns. Avec un tel spectre de longueurs d'onde, on détermine des dosages pour des composants de type alcools, sucres, et acides. Cependant, pour chacun de ces composants, la précision obtenue n'est pas la même. Notamment, les dosages des acides obtenus par spectrophotométrie dans le proche ou le moyen infrarouge ne donnent pas des résultats de grande précision. Il est donc nécessaire de faire des approximations importantes. De plus, dans le cas où certains de ces composants ne sont présents qu'en faible ou très faible quantité dans le liquide à analyser, cette méthode de dosage peut même ne pas détecter leur présence. De plus, certains composants tels que le SO2 ne peuvent même pas être dosés.

Dans l'état de la technique, on réalise également des dosages par spectrophotométrie dans l'ultraviolet et le visible. Ces dosages par spectrophotométrie dans l'ultraviolet et le visible permettent d'obtenir le dosage de composants qui ne pouvaient pas être dosés par l'intermédiaire de la spectrophotométrie dans le proche ou le moyen infrarouge.

Cependant, dans l'état de la technique, la solution proposée pour effectuer ces deux dosages est de premièrement préparer deux échantillons du liquide à analyser, et de faire passer un premier échantillon dans le spectrophotomètre des longueurs d'onde proche et moyenne infrarouge, et le deuxième échantillon dans le spectrophotomètre des longueurs d'onde ultraviolette et visible. Ces deux échantillons sont ensuite rejetés, après analyse, dans un flacon de rejet.

Mais cette solution de l'état de la technique pose un problème. En effet, elle nécessite, dans le cas où l'on souhaite effectuer les deux analyses en même temps, le prélèvement de deux échantillons distincts et donc potentiellement différents. Donc les mesures obtenues par les deux différents spectrophotomètres ne peuvent pas être correctement comparées. Dans ce cas, elle nécessite également la présence de deux systèmes d'alimentation des deux spectrophotomètres distincts et des dispositif de traitement informatique distincts.

De plus, ces méthodes de l'état de la technique posent un problème, que les dosages aient été obtenus par spectrophotométrie moyenne et proche infrarouge ou par spectrophotométrie dans l'ultraviolet ou le visible. En effet, la qualité des résultats est insuffisante, et les niveaux minimum de concentrations détectés peuvent être élevés. Alors les composants présents en faible concentration ou à l'état de trace peuvent ne pas être détectés. Or, ces composants même à l'état de traces peuvent avoir une incidence très importante sur la qualité du produit analysé. Etant donné que l'objet de ces mesures, dans une application industrielle préférée, est de permettre un jugement qualitatif du liquide, notamment si le liquide est un vin. Or avec une telle incertitude sur les résultats quantitatifs, il devient impossible de correctement baser un jugement qualitatif. L'évaluation qualitative fournie à cette occasion n'est donc pas sûre.

On connait de plus par le document WO-A-99/08115 un dispositif d'analyse correspondant à celui décrit dans le préambule de la revendication indépendante 1. L'invention a pour objet de résoudre les problèmes posés dans l'état de la technique en proposant un dispositif d'analyse comportant de préférence un dispositif d'alimentation unique du dispositif de test. En effet, le dispositif comporte un circuit d'alimentation permettant d'alimenter des bancs de test. Un premier banc de test est par exemple disposé entre une première source et un premier détecteur, spécifiques d'un spectrophotomètre à moyen infrarouges ou proches infrarouges. Alors que le deuxième banc de test est disposé entre une deuxième source et un deuxième détecteur, spécifiques d'un spectrophotomètre à longueurs d'onde dans l'ultraviolet et le visible.

Un banc de test permet de stocker le liquide à analyser pendant une période donnée pour que le premier spectrophotomètre du dispositif puisse faire une première mesure, et que en même temps voire de manière décalée dans le temps, le deuxième spectrophotomètre du dispositif puisse également faire une mesure de ce liquide. Les deux spectrophotomètres analysant des bandes de longueurs d'onde différentes, un large spectre est ainsi couvert, et exploité statistiquement simultanément.

Un spectrophotomètre à longueurs d'onde dans l'ultraviolet ou le visible, et un spectrophotomètre proche et moyen infrarouge comportent chacun des sources, des détecteurs et des bancs de test ayant des caractéristiques techniques différentes. Dans un mode de réalisation particulier le circuit d'alimentation dessert en série le premier spectrophotomètre , puis le deuxième spectrophotomètre. Dans une variante, les deux spectrophotomètres sont en parallèle, et le circuit d'alimentation prévoit de diviser l'échantillon pour alimenter chacun des bancs de test. Ainsi au cours du passage des échantillons de fluides à analyser dans le dispositif, deux spectrophotomètres peuvent effectuer le dosage sur un même échantillon des différents composants contenus dans le liquide.

L'invention présente l'avantage de ne pas générer de perte de temps, ni de nécessiter un double échantillonnage, ni une double manutention des échantillons, ni de risquer des erreurs entre les échantillons. De plus cette méthode est plus rapide. De plus, l'intérêt de l'invention est qu'elle permet par cette double analyse spectrophotométrique, une plus grande précision des dosages effectués pour les différents composants analysés dans le liquide.

L'invention concerne un dispositif d'analyse spectrophotométrique d'un fluide tel que défini dans la revendication 1.

L'invention sera mieux comprise à la lecture de la description qui suit et à l'examen des figures qui l'accompagnent. Celles-ci ne sont présentées qu'à titre indicatif et nullement limitatif de l'invention. Les figures montrent :
- Figure 1 : un schéma d'un dispositif d'analyse spectrophotométrique selon l'invention ;
- Figure 2 : un schéma d'un mode de réalisation du dispositif spectrophotométrique selon l'invention.

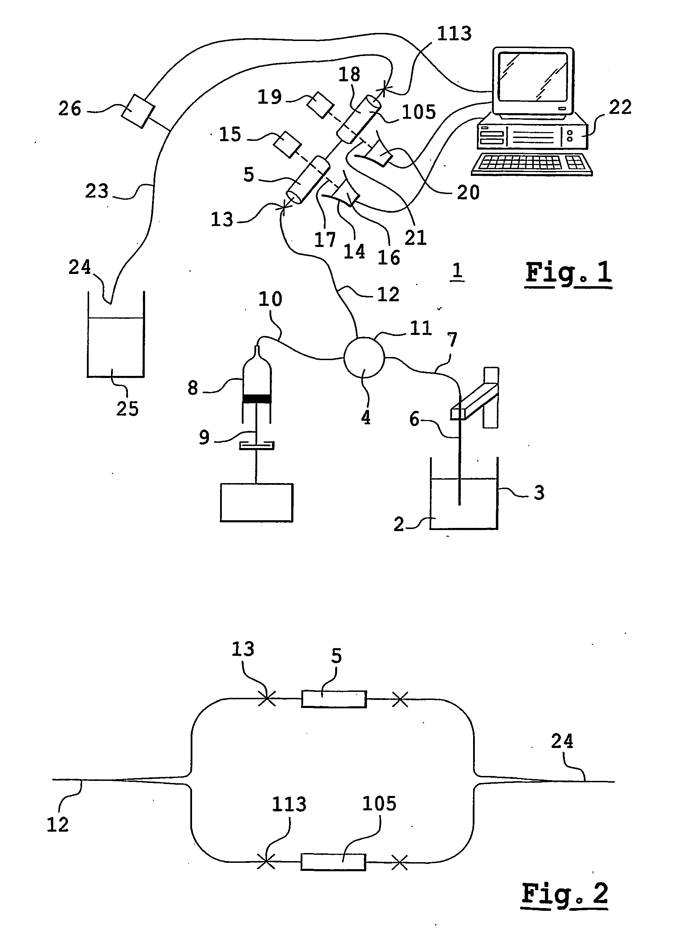

La figure 1 présente un dispositif 1 selon l'invention. Le dispositif 1 sert à analyser un fluide 2. Le fluide 2 est préférentiellement un liquide. Dans ce cas, comme représenté, il est contenu dans un réservoir 3. Ce réservoir 3 peut par exemple contenir uniquement un échantillon du liquide 2 à analyser. Dans ce cas, le réservoir 3 présente un volume intérieur faible. Le réservoir 3 peut par exemple être disposé sur un dispositif d'alimentation pouvant recevoir plusieurs réservoirs tels que 3. Le dispositif d'alimentation dessert le dispositif 1, et est par exemple rotatif.

Le dispositif 1 comporte un circuit d'alimentation 4 d'un banc de test 5 du dispositif 1. Le banc de test 5 correspond à une zone au niveau de laquelle l'analyse du fluide 2 est réalisée. Dans un mode de réalisation particulier, le circuit d'alimentation 4 comporte une pipette 6, pouvant être plongée dans le réservoir 3. Cette pipette 6 est reliée par l'intermédiaire d'un premier tube 7 à une chambre 8.

Dans la chambre 8 il est possible de créer une dépression momentanément de manière à faire venir le fluide 2 dans cette chambre 8. Dans un mode de réalisation préféré, la chambre 8 correspond à une cavité intérieure d'une seringue munie d'un piston 9. Le déplacement du piston 9 permet alors de créer momentanément des dépressions dans la chambre 8. Dans une variante, pour créer une dépression permettant de mettre le fluide 2 en mouvement, on utilise une pompe péristaltique.

Ensuite, le fluide 2 stocké dans la chambre 8 est envoyé par le biais d'un deuxième tube 10, et éventuellement d'une vanne trois voies 11 en direction du banc de test 5.

Dans ce mode de réalisation, la vanne trois voies 11 comporte une première entrée reliée au premier tube 7, une deuxième entrée reliée au deuxième tube 10 et une troisième sortie reliée au troisième tube 12, ce troisième tube 12 reliant précisément la vanne trois voies 11 au banc de test 5.

Ainsi par le biais du circuit d'alimentation 4, un flux continu et régulier du fluide à analyser 2 est créé au niveau du banc de test 5. Dans un exemple préféré, on immobilise momentanément une fraction de ce fluide à analyser 2 dans le banc de test 5. Pour bloquer le fluide 2 dans le banc de test 5, le tube 12 comporte par exemple deux électrovannes 13 disposées de part et d'autre du banc de test 5. Le flux étant régulier, dans le cas où la fermeture des deux électrovannes 13 étant simultanée, une fraction de fluide 2 est donc bloquée dans le banc de test 5. Les surpressions internes sont dé ce fait limitées. En effet, le flux est laminaire lorsque les deux électrovannes 13 sont fermées. Ainsi, lorsque le liquide 2 est analysé, il ne présente pas de mouvements microscopiques au niveau du banc de test 5.

Pour effectuer ces analyses, le dispositif 1 comporte un premier spectrophotomètre 14. Le premier spectrophotomètre 14 est disposé en vis-à-vis du banc de test 5. Notamment il comporte une première source de lumière 15 émettant en direction d'un premier détecteur 16. Ce premier détecteur 16 est disposé en vis-à-vis de l'émetteur 15 de manière à ce qu'un flux de lumière 17 émis par la source 15 traverse le banc de test 5. Cette première source de lumière 15 émet préférentiellement dans une première gamme de longueurs d'onde. Par conséquent, le banc de test 5 est réalisé avec une épaisseur et dans un matériau spécifiquement adaptés aux longueurs d'onde émises par la source 15. De même, les caractéristiques techniques du premier détecteur 16 sont adaptées.

De plus, le dispositif 1 comporte un deuxième spectrophotomètre 18. Le deuxième spectrophotomètre 18 comporte une deuxième source de lumière 19 et un deuxième détecteur 20. Le deuxième spectrophotomètre 18 est vis-à-vis d'un deuxième banc de test 105. Dans ce cas, la deuxième source de lumière 19 et le deuxième détecteur 20 sont disposés de part et d'autre du banc de test 105, de telle sorte qu'un flux de lumière 21 émis par la source 19 traverse le banc de test 105. Le banc de test 105 comporte de préférence deux électrovannes 113 de part et d'autre pour bloquer le fluide lors de l'analyse.

Ce deuxième spectrophotomètre 18 émet de la lumière dans une deuxième gamme de longueurs d'onde. A cet effet, l'épaisseur et les matériaux constituants le banc de test 105 sont également spécifiquement adaptés aux longueurs d'onde émises par la deuxième source 19. De même, les caractéristiques techniques du deuxième détecteur 20 sont spécifiquement adaptées aux longueurs d'onde émises par la deuxième source 19.

Mais dans une variante préférée, le deuxième spectrophotomètre sert à balayer un spectre plus large de longueurs d'onde de manière à obtenir un spectre d'absorbance pour des longueurs d'onde comprises dans une plus grande gamme. A cet effet, la deuxième gamme de longueurs d'onde est préférentiellement distincte de la première gamme de longueurs d'onde.

Dans un mode de réalisation préférée, le premier spectrophotomètre 14 permet d'obtenir des spectres d'absorbance pour des longueurs d'onde comprises dans les zones proche infrarouge et moyenne infrarouge, soit dans les gammes comprises entre 1,5 micron et 2,5 microns, et respectivement des gammes comprises entre 2,5 microns et 20 microns.

Dans ce cas, la première source 15 est par exemple un halogène, ou un laser ou un filament chauffé. Et le premier détecteur 16 est alors réalisé en silicium ou DTGS. La deuxième source 19 est par exemple une lampe à deutérium, ou à tungstène, alors que le deuxième détecteur 20 peut être une barrette de diodes ou des détecteurs CCD.

Ces longueurs d'onde moyenne infrarouge et proche infrarouge permettent notamment de déterminer les dosages des composants suivants : les alcools, l'éthanol, le SO2 total, le CO2, le mannitol, l'arabitol, le glycérol, le butanediol, le sorbitol, le méthyl3 Butanol 1, l'acétate d'éthyle, le mésoinositol, l'extrait sec (évaluation de la densité (degré Brix) du liquide analysé), l'azote α aminé, l'azote ammoniacal, les sucres, les sucres réducteurs, les sucres totaux, le glucose, le fructose, les acides totaux, les acides volatiles, les acides organiques, l'acide tartrique, l'acide acétique, l'acide lactique, l'acide malique, l'acide gluconique, et les ions H3O+, pour évaluer le pH.

Les longueurs d'onde moyenne infrarouge permettent notamment de déterminer les dosages des composants organiques.

Le deuxième spectrophotomètre permet d'obtenir des spectres d'absorbance pour des longueurs d'onde comprises dans la zone des ultraviolets et du visible. Il permet de balayer un spectre de longueurs d'onde compris dans une gamme comprise entre 0,1 micron et 1 micron. Ces longueurs d'onde de l'ultraviolet et du visible permettent notamment de déterminer les dosages des composants suivants : les ions H3O+, les acides, les acides totaux, les acides volatiles, l'acide acétique, l'acide tartrique, l'acide gluconique, l'acide sorbique, les polyphénols, les tannins, le SO2 libre, le SO2 total, les anthocyanes, les nitrates, l'oxygène dissous, les composants volatils.

Dans l'ultraviolet et le visible, on connaît des méthodes de référence, officielles, permettant de mesurer la densité optique (DO280), et la couleur, à partir de par exemple deux ou trois longueurs d'onde (0,420 micron, 0,520 micron, 0,620 micron).

Dans l'ensemble, ces dosages et mesures présentent un intérêt pour évaluer des caractères qualitatifs de liquides testés tels que le vin. Par exemple, pour un vin ces dosages peuvent être réalisés à différents stades de son élaboration.

Par exemple, on effectue un contrôle de maturité des raisins avant cueillette.

Puis au moment de la vendange, on vérifie l'état sanitaire du raisin et du jus qui peut en être extrait. On évalue ainsi un niveau de contamination par des bactéries de type pourritures du liquide analysé. De plus, un dosage des différents sucres du liquide permet de vérifier que le jus de macération n'a pas fait l'objet d'un enrichissement illégal à partir d'un sucre externe aux grains de raisins initiaux.

Enfin le moût en fermentation, et le vin en fin de fermentation lors de sa mise sur le marché, sont contrôlés régulièrement. Ils permettent globalement d'effectuer un contrôle de maturité et de suivre le potentiel du produit.

Notamment, la couleur, le dosage des polyphénols et des anthocyanes permettent de déterminer un indice de qualité commerciale du produit, et d'évaluer son potentiel. Ces paramètres sont principalement suivis au fur et a mesure de l'avancement du procédé d'élaboration du vin. Il permettent par exemple de connaître le savoir faire mis en oeuvre pendant le broyage des raisins, et de suivre l'évolution pendant la macération.

La largeur du spectre d'émission de longueurs d'onde couvert par le premier spectrophotomètre 14 et le deuxième spectrophotomètre 18 est de conjointement 0,1 micron à 25 microns.

Dans un mode de réalisation préféré, les spectres d'absorbance mesurés par les détecteurs respectivement 16 et 20 sont préférentiellement traités par l'intermédiaire d'un ordinateur 22. Cet ordinateur 22 permet de corréler les valeurs d'absorbance lues par chacun des deux détecteurs 16 et 20, à des spectres d'absorbance de référence correspondant respectivement à chacun de ces détecteurs, pour les différents composants à doser. Ainsi on obtient notamment une plus grande précision de dosage des différents composants acides, des polyphénols totaux, des anthocyanes, de l'acide tartrique, du SO2 libre, du SO2 total, et de l'acide sorbique présents dans le fluide 2 à analyser.

L'ordinateur 22 collecte tous les spectres obtenus par chacun des deux spectrophotomètres 14 et 18. A partir de l'ensemble des résultats d'absorbance fournis par les gammes de longueurs d'onde couvertes par les deux spectrophotomètres, on établit le dosage des composants. Les méthodes mathématiques, par exemple de PLS ou de MLR, sont appliquées, de préférence simultanément, à cet ensemble de données fournies par les spectres obtenus dans l'ultraviolet et le visible, et les spectres obtenus dans le proche et le moyen infrarouge, et non de manière séparée.

Lorsque les spectres de longueurs d'onde pouvant être émis par chacune des deux sources de lumière 15 et 19 ont été parcourus, alors, on prévoit d'ouvrir les électrovannes 13 et 113 de manière à libérer le fluide 2 désormais analysé. Pour assurer la sortie du fluide 2, on peut prévoir de pousser plus encore le piston 9 de manière à pousser définitivement l'intégralité du liquide dans un tube de sortie 23. Mais dans une variante, on peut également prévoir que à une extrémité 24 de ce tube de sortie 23, une pompe aspirante soit prévue. Dans un mode de réalisation préféré, le tube de sortie 23 débouche dans un récipient de déchets 25. Le récipient de déchets 25 reçoit les différents échantillons analysés issus de fluides à analyser tels que 2.

Dans le cas où les spectrophotomètres sont disposés en série (Figure 1), il y a un unique tube de sortie 23. Alors que dans le cas où ils sont disposés en parallèles (Figure 2), soit chaque banc de test est relié à son propre tube de sortie, chaque tube conduisant vers un récipient de déchets ; soit les deux tubes se rejoignent pour former une même extrémité 24.

Dans un mode de réalisation particulier, on prévoit que le dispositif d'analyse 1 comporte de plus une sonde 26 pour mesurer la conductivité du fluide à analyser 2. Cette sonde 26 est préférentiellement également reliée à l'ordinateur 22. Les données obtenues par la sonde 26 permettent de déduire notamment le potentiel oxydoréducteur du fluide 2. Cette sonde 26 sert uniquement pour l'analyse de fluides 2 de type liquide.

Dans le cas où le fluide à analyser 2 est un liquide sanguin d'origine humaine ou animale, on profite notamment des deux spectrophotomètres 14 et 18 pour doser les composants suivants : le glucose, le cholestérol, la créatine, les phosphatases, les transaminases GOT et GPT, l'urée, l'acide urique, les phospholipides, les protéines totales, HDL, LDL, les lipides totaux, les triglycérides et les gamma GT.

## Revendications

1. Dispositif (1) d'analyse spectrophotométrique d'un fluide (2) comportant un premier spectrophotomètre (14) et un deuxième spectrophotomètre (18), les spectrophotomètres comportant chacun respectivement une source de lumière (15, 19) et un détecteur (16, 20) disposés de part et d'autre de respectivement un premier banc de test (5) et un deuxième banc de test (105), une première source (15) de lumière émettant dans une première gamme de longueurs d'onde en direction du premier banc de test et une deuxième source (19) de lumière émettant dans une deuxième gamme de longueurs d'onde, différente de la première gamme de longueurs d'onde, en direction du deuxième banc de test, **caractérisé en ce qu'**il comporte un ordinateur (22) arrangé pour traiter et corréler des valeurs d'absorbance fournies par l'un des spectrophotomètres avec des valeurs d'absorbances fournies par l'autre des spectrophotomètres.

2. Dispositif selon la revendication 1 **caractérisé en ce que** la première gamme de longueurs d'onde correspond à une gamme de longueurs d'onde moyenne infrarouge et ou proche infrarouge

3. Dispositif selon l'une des revendications 1 à 2 **caractérisé en ce que** la deuxième gamme de longueurs d'ondes correspond à une gamme de longueurs d'onde de l'ultraviolet ou du visible.

4. Dispositif selon l'une des revendications 1 à 3 **caractérisé en ce que** la première source de lumière et la deuxième source de lumière peuvent présenter conjointement un spectre d'émission de longueurs d'onde compris entre 0,1 micromètres et 20 micromètres.

5. Dispositif selon l'une des revendications 1 à 4 **caractérisé en ce qu'**il comporte une sonde (26) pour mesurer la conductivité du fluide.

6. Dispositif selon l'une des revendications 1 à 5 **caractérisé en ce que** les deux spectrophotomètres sont disposés en série.

7. Dispositif selon l'une des revendications 1 à 6 **caractérisé en ce que** les deux spectrophotomètres sont disposés en parallèle.

8. Dispositif selon l'une des revendications 1 à 6 **caractérisé en ce que** le liquide à analyser est un liquide aqueux alcoolique ou non, ou un liquide humain ou animal.

## Claims

1. Device (1) for spectrophotometric analysis of a fluid (2), comprising a first spectrophotometer (14) and a second spectrophotometer (18), the spectrophotometers each comprising a light source (15, 19) and a detector (16, 20) which are arranged at one side and the other of a first test bed (5) and a second test bed (105), respectively, a first light source (15) emitting in a first range of wavelengths in the direction of the first test bed and a second light source (19) emitting in a second range of wavelengths which is different from the first range of wavelengths in the direction of the second test bed, **characterised in that** it comprises a processor (22) which is arranged in order to process and correlate absorption values which are provided by one of the spectrophotometers with absorption values provided by the other of the spectrophotometers.

2. Device according to claim 1, **characterised in that** the first range of wavelengths corresponds to a range of wavelengths of medium-infrared or near-infrared.

3. Device according to either claim 1 or claim 2, **characterised in that** the second range of wavelengths corresponds to a range of wavelengths of ultraviolet or visible light.

4. Device according to any one of claims 1 to 3, **characterised in that** the first light source and the second light source may jointly have an emission spectrum of wavelengths between 0.1 micrometres and 20 micrometres.

5. Device according to any one of claims 1 to 4, **characterised in that** it comprises a probe (26) for measuring the conductivity of the fluid.

6. Device according to any one of claims 1 to 5, **characterised in that** the two spectrophotometers are arranged in series.

7. Device according to any one of claims 1 to 6, **characterised in that** the two spectrophotometers are arranged in parallel.

8. Device according to any one of claims 1 to 6, **characterised in that** the liquid to be analysed is an aqueous liquid which may or may not be alcoholic, or a human or animal liquid.

## Patentansprüche

1. Vorrichtung (1) zur spektrophotometrischen Analyse einer Flüssigkeit (2), die ein erstes Spektralphotometer (14) und ein zweites Spektralphotometer (18) umfasst, wobei jedes Spektralphotometer jeweils eine Lichtquelle (15, 19) und einen Detektor (16, 20) umfasst, die auf beiden Seiten von einem ersten Prüfstand (5) beziehungsweise einem zweiten Prüfstand (105) angeordnet sind, wobei eine erste Lichtquelle (15) in einem ersten Wellenlängenbereich in Richtung des ersten Prüfstands emittiert, und eine zweite Lichtquelle (19) in einem zweiten Wellenlängenbereich, der sich vom ersten Wellenlängenbereich unterscheidet, in Richtung des zweiten Prüfstands emittiert, **dadurch gekennzeichnet, dass** sie einen Computer (22) umfasst, der so angeordnet ist, dass er die Extinktionswerte, die von einem der Spektralphotometer bereitgestellt werden, verarbeitet und mit den Extinktionswerten, die von dem anderen Spektralphotometer bereitgestellt werden, korreliert.

2. Vorrichtung nach Anspruch 1, **dadurch gekennzeichnet, dass** der erste Wellenlängenbereich einem Wellenlängenbereich des mittleren oder nahen Infrarots entspricht.

3. Vorrichtung nach einem der Ansprüche 1 bis 2, **dadurch gekennzeichnet, dass** der zweite Wellenlängenbereich einem Wellenlängenbereich des ultravioletten oder sichtbaren Bereichs entspricht.

4. Vorrichtung nach einem der Ansprüche 1 bis 3, **dadurch gekennzeichnet, dass** die erste Lichtquelle und die zweite Lichtquelle gemeinsam ein Emissionsspektrum mit einer Wellenlänge zwischen 0,1 Mikrometern und 20 Mikrometern aufweisen können.

5. Vorrichtung nach einem der Ansprüche 1 bis 4, **dadurch gekennzeichnet, dass** sie eine Sonde (26) zum Messen der Leitfähigkeit der Flüssigkeit umfasst.

6. Vorrichtung nach einem der Ansprüche 1 bis 5, **dadurch gekennzeichnet, dass** die beiden Spektralphotometer in Reihe angeordnet sind.

7. Vorrichtung nach einem der Ansprüche 1 bis 6, **dadurch gekennzeichnet, dass** die beiden Spektralphotometer parallel angeordnet sind.

8. Vorrichtung nach einem der Ansprüche 1 bis 6, **dadurch gekennzeichnet, dass** die zu untersuchende Flüssigkeit eine wässrige, alkoholische oder nicht alkoholische Flüssigkeit oder eine Flüssigkeit menschlichen oder tierischen Ursprungs ist.
